# EUROPÄISCHE PATENTSCHRIFT

(11) **EP 2 914 309 B1**
(45) Veröffentlichungstag und Bekanntmachung des Hinweises auf die Patenterteilung: **17.07.2019**
(21) Anmeldenummer: 13798564.4
(22) Anmeldetag: 30.10.2013
(51) Int. Cl.: A61L 31/02, A61L 31/10, A61L 31/16, A61F 2/82, A61F 2/915

(54) **BIORESORBIERBARER STENT AUS EINEM VERBUNDMATERIAL**
BIORESORBABLE STENT CONSISTING OF A COMPOSITE MATERIAL
STENT BIORÉSORBABLE CONSTITUÉ D'UN MATÉRIAU COMPOSITE

(30) Priorität: 30.10.2012 DE 102012021187
(43) Veröffentlichungstag der Anmeldung: 09.09.2015
(73) Patentinhaber: Nißl, Thomas, 37318 Mackenrode (DE)
(72) Erfinder: NIßL, Thomas, 37318 Mackenrode (DE)
(74) Vertreter: Thiel, Christian
(86) Internationale Anmeldenummer: PCT/EP2013/003263
(87) Internationale Veröffentlichungsnummer: WO 2014/067656

(56) Entgegenhaltungen:
- EP-A1- 1 842 507
- EP-A1- 2 018 834
- EP-A1- 2 656 818
- EP-A2- 2 329 853
- DE-A1- 19 731 021
- US-A1- 2008 082 162

## Beschreibung

Die Erfindung betrifft einen bioresorbierbaren Stent aus einem Metall/Polymer-Verbund mit einer Vielzahl von Ringsegmenten und Distanzelementen zwischen Ringsegmenten, wobei der Stent ein Gerüst aus einem bioresorbierbaren Metall mit einer Beschichtung aus einem bioresorbierbaren Polymer aufweist.

Stents werden vielfach zur Beseitigung von Engpässen in Körperdukten eingesetzt, beispielsweise vaskulären Engpässen aber auch solchen in der Speiseröhre, in den Gallengängen oder in Darmbereich. Häufigster Einsatzgrund ist eine Verengung der Herzkranzgefäße durch Arteriosklerose. Im folgendem wird die Erfindung am Beispiel eines Koronarstents beschrieben; es sei aber darauf hingewiesen, dass die erfindungsgemäßen Stents auch für nichtvaskuläre Zwecke eingesetzt werden können.

Eine Arteriosklerose kann eine Reihe von Ursachen haben, beispielsweise auf eine vaskuläre Verletzung oder Infektion zurückgehen, bei der es zur Ausbildung von Ablagerungen oder zu einer Hyperproliferation der vaskulären Epithelzellen kommt. In der Folge kann es zu einer mehr oder wenig starken lokalen Verengung des betroffenen Blutgefäßes kommen, bis hin zum Gefäßverschluss und zum Absterben des von dem jeweiligen Gefäß versorgten Gewebes. Im Koronarbereich führt eine solche Verstopfung zum Herzinfarkt.

Gefäßverengungen im Bereich der Herzkranzgefäße können auf verschiedene Art und Weise behandelt werden. Zum einem besteht die Möglichkeit einer Bypassoperation, die mit einem mehr oder weniger schweren operativen Eingriff verbunden ist. Endovaskulär können Gefäßverengungen durch Ballondilation beseitigt werden, bei der die verengte Stelle des Gefäßes mit Hilfe eines hydraulisch aufblasbaren Ballons dilatiert wird. Die Gefäßdilation ist zumeist auch mit der Platzierung eines Stents verbunden, bei der der Stent auf den Ballon aufgekrimpt ist und mit diesem aufgeweitet und in der verengten Stelle des Gefäßes platziert wird. Der Stent stützt das Gewebe und hält im Idealfall das Gefäß offen. Diese auch als "perkutane transluminale coronare Angioplastie" (PTCA) und "stenting" bezeichnete Behandlungsweise ist für den Patienten erheblich schonender als eine operative Behandlung der Gefäßverengung.

Die mit Hilfe von aufblasbaren Ballons gesetzten Stents werden mit einem erheblichen Druck an die Gefäßwand gepresst, meist mit deutlich mehr als 10⁶ Pa (10 bar). Die dadurch auf die Gefäßwand ausgeübte Druckbelastung kann zu Schäden führen, die zu erneuter Hyperproliferation der Epithelzellen und zu einer Anlagerung von Gewebe an dem Stent führt. Eine Restenose ist die Folge, d. h. eine erneute Gefäßverengung.

Solchen restenoischen Gefäßverengung versucht man auf mehreren Wegen zu begegnen, die bislang aber nur zum Teilerfolg geführt haben. Zur Verminderung der Gefäßschäden versucht man zum einen, die Druckbelastung beim Setzen des Stents gering zu halten. Da aber ein gewisser Mindestdruck aufgewandt werden muss, um das Gefäß zu erweitern, sind diesem Ansatz Grenzen gesetzt.

Ein anderer weitverbreiteter Ansatz ist die sorgfältige Glättung der Stentoberfläche durch Polierverfahren, um Verletzungen durch Grate und Unebenheiten zu vermeiden. Zu diesem Zweck werden Stents mechanisch oder elektrochemisch geglättet. Auch diesem Ansatz sind Grenzen gesetzt, da das Aufkrimpen eines Stents auf einen Ballon und die anschließende Erweiterung des Stents beim Platzieren zu Unregelmäßigkeiten an der Oberfläche führen. Zudem bleibt der hydraulische Druck bei der Stenterweiterung.

Hinzu kommt, dass insbesondere im koronaren Bereich die Gefäße in Muskelgewebe eingebettet sind. Wenn das Muskelgewebe arbeitet, kommt es zu einer ständigen Belastung der Gefäßwand an den gestenteten Stellen durch mechanische Reibung. Auch diese Reizung kann zur Restenose beitragen.

Um die Restenosegefahr durch prolifertives Zellwachstum zu begrenzen, werden proliferartionshemmende Medikamente eingesetzt, beispielsweise Paclitaxel oder Rapamycin. Diese Medikamente können beispielsweise durch die Verwendung damit beschichteter Ballone auf die Gefäßwand aufgebracht werden. Eine andere Möglichkeit ist die Beschichtung von Stents mit solchen proliferationshemmenden Mitteln. Eingesetzt werden beispielsweise medikamentenhaltige Beschichtungen aus biokompatiblen Polymeren, die im Laufe der Zeit vom Körper absorbiert werden. Diese Beschichtungen setzt das Medikament über einen mehr oder weniger langen Zeitraum frei.

Die Praxis hat gezeigt, dass die Restenosegefahr in den ersten Tagen und Wochen nach der Gefäßdilatation am größten ist. Insoweit kommt es darauf an, ein poliferationshemmendes Medikament vor allem in dieser Zeit bereitzustellen.

Die Praxis hat ferner gezeigt, dass nach der Platzierung eines Stents die über die Dilatation bewirkte Gefäßerweiterung sich im Laufe der Zeit stabilisiert. Dies bedeutet, dass der Stent nach einer gewissen Zeit überflüssig wird, d. h. eigentlich entfernt werden könnte. Die Entfernung von Stents aus dem Gefäßsystem ist aber problematisch, einmal wegen der engen Anpassung der Stentoberfläche an die Gefäßinnenwand und zum anderen wegen der vielfach beobachteten Einbeziehung des Stents in den Zellverbund an der Gefäßoberfläche. Weiterhin ist es ein Problem, einen Stent mit einem Extraktionsgerät zu greifen

Diese Erkenntnis hat dazu geführt, dass selbstauflösende (bioresorbierbare) Stents entwickelt wurden. Materialien sind auf der einen Seite Biopolymere, die aber häufig nicht die nötige Radialkraft mit sich bringen, um die Gefäßerweiterung aufrechtzuerhalten. Es wurde ferner gefunden, dass einige Metalle und Metalllegierungen eine begrenzte Haltbarkeit im Körper aufweisen und sich im Laufe der Zeit, abhängig vom Material und der Materialstärke, auflösen. Diese Beobachtung wurde bei Reineisen gemacht, aber auch bei Magnesium und einigen Magnesiumlegierungen, etwa solchen, die geringe Mengen Mangan und/oder Seltenerdmetalle enthalten. Solche Materialien sind vielfach, auch in der Patentliteratur, beschrieben.

Im Allgemeinen wird die Zeit, die ein Stent aus einem bioresorbierbaren Teil zur Auflösung benötigt, als zu lang empfunden. Andererseits kann in der Regel die Materialstärke nicht beliebig vermindert werden, schon aus fertigungstechnischen und Statikgründen. Eine Verminderung der Wandstärke und/oder Stegbreite herkömmlicher Stents führt zu einer Verminderung der Radialkraft und steht damit dem eigentlichen Zweck des Stents, das Gefäß aufzuweiten und in dem Zustand zu halten, entgegen.

Aus der WO 2012/083796 A (EP 2 656 818 A1) ist ein sich schnell auflösender Metallstent bekannt, der geschlitzte Stege aufweisen kann und eine ausreichende Stabilität und Radialkraft aufweist.

Aufgabe der Erfindung ist deshalb die Bereitstellung eines Stents, der bei hinreichender Stabilität und Radialkraft eine deutlich verminderte Auflösungszeit im Körper eines Patienten hat.

Diese Aufgabe wird mit einem Stent der eingangs bezeichneten Art gelöst, bei dem das Metallgerüst eine Vielzahl durchgehend geschlitzter Stege aufweist, die zur Verstärkung mit dem bioresorbierbaren Polymer gefüllt sind und ein Verbundprofil mit von zwei Metallwangen eingefasstem Kunststoffkern ausbilden, so dass das Verbundprofil die zur Erfüllung der statischen Funktion benötigte Festigkeit und Radialkraft erhält, wobei der Polymerkern der Stege 30 % bis 70 % der Stegbreite ausmacht.

Die erfindungsgemäßen bioresorbierbaren Stents bestehen aus einer Vielzahl von herkömmlich gestalteten Ringsegmenten und Distanzelementen zwischen den Ringsegmenten. Die Ringsegmente können beispielsweise übliche zickzackförmige oder mäandrierende Stegabfolgen sein, sind aber vorzugsweise rautenförmig gestaltet. In diesen Segmenten sind bei denen die Rautenelemente innerhalb des Ringsegments über ihre Spitzen in Knotenpunkten verbunden sind.

Als Distanzelemente zwischen benachbarten Ringsegmenten kommen üblicherweise verwandte Distanzelemente in Stegform in Frage, die beispielsweise an den Spitzen oder in den Eintiefungen der zickzackförmigen, mäandrierenden oder rautenförmigen Ringelemente ansetzen und gegebenenfalls S-förmige oder spiralförmige Abschnitte aufweisen. Solche Distanzelemente sind vielfach beschrieben.

Der erfindungsgemäße Stent weist ein Gerüst aus einem bioresobierbaren Metall mit einer Beschichtung aus einem bioresobierbaren Polymer auf. Als bioresorbierbares Metall kommen insbesondere Eisen, Magnesium und Magnesiumlegierungen in Frage. Als bioresobierbare Polymere sind insbesondere die Resomere® der Firma Böhringer zu nennen, etwa Polymere auf Basis von Lactid- und Glycolideinheiten sowie Copolymere davon. Zu nennen sind aber auch natürliche Polymere, beispielsweise Chitin oder auch Schellack.

Im erfindungsgemäßen Stent ist das Metallgerüst so "ausgedünnt", dass es allein die statischen Funktionen nicht mehr erfüllen kann. Erst im Verbund mit dem bioresorbierbaren Polymer wird eine hinreichende Festigkeit und Radialkraft erreicht. Dazu ist das Metallgerüst so ausgebildet, dass es eine Vielzahl geschlitzter Stege aufweist, die zur Verstärkung mit dem Biopolymer gefüllt sind und ein Verbundprofil mit einem von zwei Metallwangen eingefassten Kunststoffkern ausbilden.

Insbesondere besteht das Metallgerüst insgesamt aus geschlitzten Stegen. Die Stege sind durchgehend geschlitzt. Es versteht sich, dass die Wangen der geschlitzten Stege über stabilisierende Brücken verbunden sein müssen, die zueinander beabstandet sind und insbesondere an Wendepunkten und Knotenpunkten auftreten, und den Zusammenhalt und die Integrität zusichern.

Im erfindungsgemäßen Stent sind die Schlitze mit dem bioresorbierbaren Polymer gefüllt, so dass ein Sandwichprofil entsteht mit zwei aussenliegenden Metallschichten oder -wangen und einer dazwischen liegenden Polymerschicht.

Vorzugsweise haben die Stege des erfindungsgemäßen Stents einen in etwa quadratischen Querschnitt.

Die Stents werden auf eine an und für sich bekannte Art und Weise aus Rohren des entsprechenden Materials mit Hilfe eines Lasers geschnitten. Nach der üblichen Behandlung und Ausrüstung werden sie auf einen Ballon gekrimpt. Dieser Ballon wird in das Gefäß eingeführt und hydraulisch aufgeblasen, was den Stent aufweitet und an die Wand des entsprechenden Gefäßes drückt. Dieser Aufweitungsprozess führt zu einer erheblichen mechanischen Belastung der Gerüststruktur.

Erfindungsgemäß macht der Kunststoffkern zwischen den Metallwangen 30 bis 70 Prozent der gesamten Stegbreite aus. Die Stege selbst haben eine Stärke von 60 bis 200 µm und insbesondere von etwa 80 bis 140 µm, was der Wandstärke der Rohre entspricht, aus dem die erfindungsgemäßen Stents geschnitten werden. Die Brücken, die die Metallwangen der Stege miteinander verbinden, haben in etwa die gleiche Stärke wie die Metallwangen.

Im Bereich der Knotenpunkte des erfindungsgemäßen Stents, d. h. dort, wo sich mehrere Stege treffen, kann es sinnvoll sein, Durchbrechungen vorzusehen, um die Metallmasse in diesen Punkten zu begrenzen und die Oberfläche groß zu halten. Das Auflösungsverfahren der Metallteile des Stents hängt entscheidend vom Verhältnis Oberfläche/Volumen ab. Zweckmäßig ist es, die Wandstärke einzelner Metallelemente auf die durchschnittliche Wandstärke der Metallwangen der Stege zu begrenzen. Dies gilt auch für die Stärke der Brücken zwischen den Metallwangen. Bei den Distanzelementen kann allerdings die Stegbreite geringer ausfallen, da hier eine größere Flexibilität gefordert ist.

Wie bereits festgehalten, ist der bevorzugte metallische Werkstoff eine Magnesiumlegierung mit einem geringen Gehalt an Mangan und Seltenerdmetall. Bevorzugt sind Mangangehalte im Bereich von 1 bis 3 Gewichtsprozent und Seltenerdmetallgehalte von 0,5 bis 3 Gewichtsprozent. Bevorzugtes Seltenerdmetall ist Cer, daneben auch Neodym. Es hat sich herausgestellt, dass diese Legierungen eine außerordentlich hohe Festigkeit aufweisen. Zudem sind manganhaltige Magnesiumlegierungen unbedenklich und haben eine geringere Tendenz, bei der Auflösung Wasserstoff freizusetzen.

Die erfindungsgemäßen Stents können innerhalb ihrer Polymeranteile ein Arzneimittel enthalten. Hierzu wird insbesondere auch auf die einschlägige Literatur verwiesen. Beispielsweise sind solche Arzneimittel proliferationshemmende, entzündungshemmende und antibiotische Arzneimittel. In erster Linie kommen Rapamycin und Paclitaxel als proliferationshemmende Arzneimittel in Frage. Diese Arzneimittel können sowohl in der Beschichtung enthalten sein als auch in den gesamten Polymeranteilen und werden im Rahmen der Auflösung der Polymeranteile freigesetzt und an die Gefäßwand abgegeben.

Gemäß einer besonderen Ausführungsform ist das zum Einsatz kommende Arzneimittel im Wesentlichen nur in der äußeren Beschichtung vorhanden, d. h. nach Auffüllung der Durchbrechungen oder Schlitze zwischen den Metallwangen und einer Grundschicht wird in einem gesonderten Sprühverfahren Polymer mit einem definierten Arzneimittelgehalt auf die Oberfläche in einem oder mehreren Arbeitsgängen aufgesprüht.

Die erfindungsgemäßen Stents werden auf eine übliche Art und Weise hergestellt. Der Stentkörper wird in der Regel aus einem Rohr geschnitten und anschließend zur Abrundung der Kanten elektropoliert. Der elektropolierte Gerüstkörper wird anschließend durch Tauch- und/oder Sprühbeschichtung mit dem Polymer versetzt, der dabei auch in die Eintiefungen/Schlitze eindringt und diese ausfüllt.

Es versteht sich, dass eine entsprechende Gerüststruktur auch auf Stents angewandt werden kann, deren Metallkörper nicht bioresorbierbar ist, beispielsweise aus Nitinol oder einem medizinischen nicht resorbierbaren Stahl besteht. In diesem Fall kann das zum Einsatz kommende Polymer bioresorbierbar oder dauerhaft sein. Im Falle der Kombination eines nicht resorbierbaren Metalls mit einem bioresobierbaren Polymer verbleibt nach einer gewissen Zeit lediglich das Metallgerüst im Körper.

Der Vorteil der erfindungsgemäßen Gerüststruktur auch bei nicht resobierbaren Metallgerüsten liegt in der Kraftübertragung auf die Gefäßwand. Der geringere Metallanteil führt zu einer geringeren Kontaktfläche der Metallwangen mit der Gefäßwand und durch das spezielle Design zu einer gleichmäßigen Druckverteilung auf die Gefäßwand und, trotz geringerem Materialeinsatz, zu einer ausreichenden Radialkraft. Die filigranere Struktur des Metallgerüsts erlaubt ein schnelles und problemloses Einwachsen in die Gefäßwand.

Es versteht sich ferner, dass das Metallgerüst auch durch ein aus einem entsprechenden Rohr geschnittenes Kunststoffgerüst ersetzt werden kann, wobei der Kunststoff sowohl dauerhaft als auch bioresorbierbar sein kann. Voraussetzung ist, dass das Kunststoffmaterial eine hinreichende Festigkeit und Radialkraft bei der Expansion entwickelt. Derartige Kunststoffe sind beschrieben. In diesem Fall gibt sich eine festere Gerüststruktur aus einem ersten Kunststoffmaterial mit einer sandwichartigen Füllung eines zweiten Polymers, die den zuvor für den resorbierbaren Stent beschriebenen Profilverbund ausbilden.

Die Erfindung wird durch die beigefügten Abbildungen näher erläutert. Es zeigen:
- Abbildung 1: einen erfindungsgemäßen Stent in abgewickelter Form und
- Abbildung 2: einen Stegquerschnitt eines erfindungsgemäßen Stents und
- Abbildung 3: eine Variante des Stents von Abbildung 1.

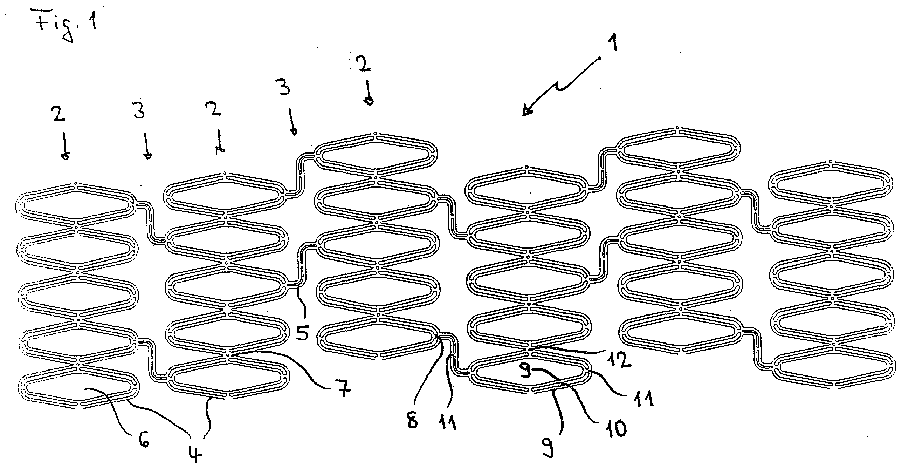

Der Stent 1 gemäß Abbildung 1 besteht aus einer Mehrzahl aus Ringsegmenten 2 und Distanzelementen 3 zwischen aneinandergrenzenden Ringsegmenten 2. Im dargestellten Fall besteht jedes Ringsegment aus fünf Rautenelementen 6, die über Knotenpunkte 7 miteinander verbunden sind, wobei die einzelnen Rauten 6 vom jeweils vier Stegen 4 gebildet und begrenzt werden.

Die Ringsegmente 2 werden über Distanzelemente 3 miteinander verbunden. Die Distanzelemente 3 werden von Stegen 5 gebildet, die an seitlichen Eckpunkten der Rauten 6 in Knotenpunkten 8 ansetzen. Die Distanzelemente 3 sind jeweils stufenförmig ausgeführt und dienen dazu, die bei der Aufweitung des Stents erfolgte Längenkontraktion durch eine Streckung aufzufangen. Des Weiteren verleihen sie dem Stent über seine Länge Flexibilität, so dass er auch durch gewundene Gefäßgänge transportiert werden kann. Die einzelnen Stege 4 der Rauten 6 und 5 der Distanzelemente 3 sind geschlitzt ausgeführt, d. h. sie bestehen aus zwei Wangen 9 mit einem dazwischen angebrachten Schlitz 10, der im fertigen Stent mit der Polymermasse gefüllt ist. Die Schlitze 10 werden seitlich durch Brücken 11 begrenzt, die in der Regel die gleiche Stärke haben wie die Wangen 9. Die Brücken 11 befinden sich insbesondere in den Knotenpunkten, Wendepunkten und bei der Aufweitung belasteten Stellen des Stents 1 und dienen der Sicherung der Verbundstruktur.

Im Bereich der Knotenpunkte 7 zwischen zwei Rautenelementen eines Ringsegments befindet sich eine Durchbrechung 12, die allein der Vergrößerung der Oberfläche und der Vermeidung von größeren Metallagglomerationen dient, welche bei der Auflösung der Metallstruktur unerwünscht große Partikel bilden könnten.

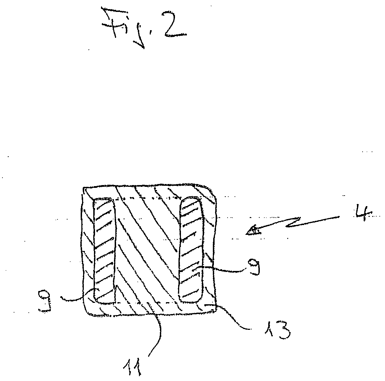

Abbildung 2 zeigt einen Steg eines erfindungsgemäßen Stents im Querschnitt mit den beiden Metallwangen 9 und der die Metallwangen umgebenden und den Zwischenraum zwischen den Metallwangen ausfüllenden Polymermasse 13. Die beiden gestrichelten Linien deuten die Brückenverbindung 11 zwischen den Wangen 9 an, die in definierten Abständen ausgebildet ist und der Stabilität dient. Die Metallwangen 9 und die dazwischenliegende Polymermasse 13 bilden den erfindungsgemäßen Profilverbund aus, der die Stabilität und Radialkraft des Stents sicherstellt. Weder das Metallgerüst allein noch die Polymermasse alleine wären geeignet, einen funktionsfähigen Stent bereitzustellen.

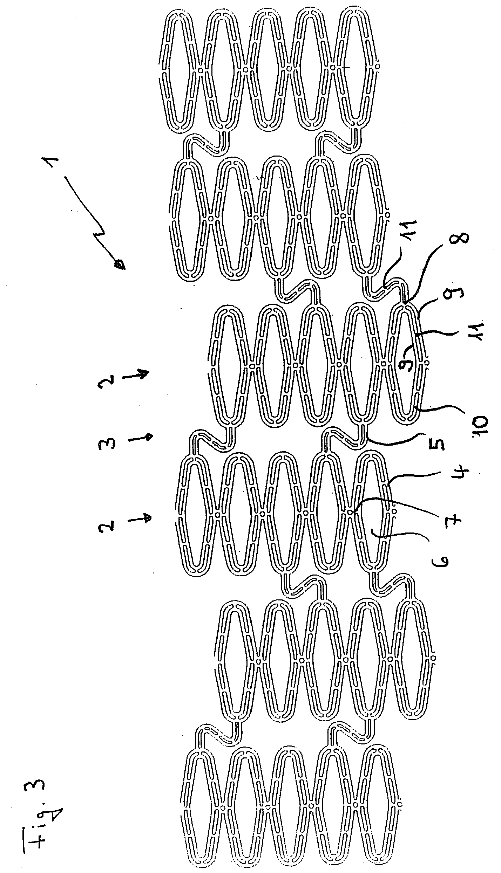

Abbildung 3 zeigt eine Variante des Stents von Abbildung 1, bei dem die zwischen den Wangen 9 befindliche Durchbrechung oder Eintiefung kürzer gestaltet ist und auch auf der Steglänge durch Brücken 11 unterbrochen ist. Der Stent erhält dadurch eine größere Stabilität und Versteifung. Gleichzeitig sind die zwischen den Ringsegmenten 2 befindlichen Distanzelemente 3 stärker S-förmig gestaltet, was dazu führt, dass sich diese Distanzelemente 3 bei der Expansion des Stents stärker strecken können und damit der Längenverkürzung entgegenwirken.

## Patentansprüche

1. Bioresorbierbarer Stent aus einem Metall/Polymer-Verbund mit einer Vielzahl von Ringsegmenten (2) und Distanzelementen (3) zwischen Ringsegmenten (2), wobei der Stent (1) ein Gerüst aus einem bioresorbierbaren Metall mit einer Füllung (13) aus einem bioresorbierbaren Polymer aufweist, **dadurch gekennzeichnet, dass** das Metallgerüst eine Vielzahl durchgehend geschlitzter Stege (4) aufweist, die zur Verstärkung mit dem Polymer (13) gefüllt sind und ein Verbundprofil mit von zwei Metallwangen (9) eingefasstem Polymerkern (13) ausbilden, so dass das Verbundprofil die zur Erfüllung der statischen Funktion benötigte Festigkeit und Radialkraft erhält, wobei der Polymerkern (13) der Stege (4) 30 % bis 70 % der Stegbreite ausmacht.

2. Stent nach Anspruch 1, **dadurch gekennzeichnet, dass** die Stege (4) zu einander beabstandete stabilisierende Brücken (11) zwischen den Wangen (9) aufweisen.

3. Stent nach Anspruch 2, **dadurch gekennzeichnet, dass** die Brücken (11) an Wendepunkten und/oder Knotenpunkten (7, 8) angeordnet sind.

4. Stent nach einem der vorstehenden Ansprüche mit einem quadratischen Querschnitt der Stege (4).

5. Stent nach einem der vorstehenden Ansprüche, **dadurch gekennzeichnet, dass** die Stegstärke im Bereich von 60 bis 200 µm liegt.

6. Stent nach einem der vorstehenden Ansprüche, **dadurch gekennzeichnet, dass** die Ringsegmente (2) von Zickzack-, Mäander- oder Rautenelementen (6) gebildet sind.

7. Stent nach Anspruch 6, **dadurch gekennzeichnet, dass** die Rautenelemente (6) eines Ringsegments (2) über Knotenpunkte (7) miteinander verbunden sind.

8. Stent nach Anspruch 7, **dadurch gekennzeichnet, dass** die Rautenelemente (6) aneinander grenzender Ringsegmente (2) an den Eckpunkten über Distanzelemente (7) miteinander verbunden sind.

9. Stent nach Anspruch 7 oder 8, **dadurch gekennzeichnet, dass** die Knotenpunkte durchbrochen sind.

10. Stent nach einem der vorstehenden Ansprüche, **dadurch gekennzeichnet, dass** das Metall eine Magnesiumlegierung oder Reineisen ist.

11. Stent nach Anspruch 10, **dadurch gekennzeichnet, dass** das Magnesium mit Mangan und einem Seltenerdmetall legiert ist.

12. Stent nach einem der vorstehenden Ansprüche, **dadurch gekennzeichnet, dass** das bioresorbierbare Polymer ein synthetisches oder natürliches Polymer ist.

13. Stent nach Anspruch 12, **dadurch gekennzeichnet, dass** das Polymer ein Polylactid, Polyglycolid oder ein Mischpolymer mit Lactid- und Glycolideinheiten ist.

14. Stent nach Anspruch 12, **dadurch gekennzeichnet, dass** das Polymer eine Biopolymer ist.

15. Stent nach Anspruch 14, **dadurch gekennzeichnet, dass** das Biopolymer Chitin ist.

16. Stent nach einem der vorstehenden Ansprüche, **dadurch gekennzeichnet, dass** das Polymer ein Arzneimittel enthält.

## Claims

1. Bioresorbable stent from a metal/polymer composite having a multiplicity of ring segments (2) and spacer segments (3) between ring segments (2), wherein the stent (1) has a framework from a bioresorbable metal having a filling (13) from a bioresorbable polymer, **characterized in that** the metal framework has a multiplicity of continuously slotted webs (4) which for reinforcement are filled with the polymer (13) and configure a composite profile having a polymer core (13) that is bordered by two metal jowls (9) such that the composite profile is imparted the strength and radial force required for fulfilling the static function, wherein the polymer core (13) of the webs (4) accounts for 30% to 70% of the web width.

2. Stent according to Claim 1, **characterized in that** the webs (4) between the jowls (9) have mutually spaced apart stabilizing bridges (11).

3. Stent according to Claim 2, **characterized in that** the bridges (11) are disposed on reversal points and/or nodal points (7, 8).

4. Stent according to one of the preceding claims, having a square cross section of the webs (4).

5. Stent according to one of the preceding claims, **characterized in that** the web thickness is in the range from 60 to 200 µm.

6. Sent according to one of the preceding claims, **characterized in that** the ring segments (2) are formed by zigzag-shaped, meander-shaped, or diamond-shaped elements (6).

7. Stent according to Claim 6, **characterized in that** the diamond-shaped elements (6) of a ring segment (2) are connected to one another by nodal points (7) .

8. Stent according to Claim 7, **characterized in that** the diamond-shaped elements (6) of mutually adjacent ring segments (2) at the corner points are connected to one another by spacer elements (7).

9. Stent according to Claim 7 or 8, **characterized in that** the nodal points are penetrated.

10. Stent according to one of the preceding claims, **characterized in that** the metal is a magnesium alloy or pure iron.

11. Stent according to Claim 10, **characterized in that** the magnesium is alloyed with manganese and a rare-earth metal.

12. Stent according to one of the preceding claims, **characterized in that** the bioresorbable polymer is a synthetic or natural polymer.

13. Stent according to Claim 12, **characterized in that** the polymer is a polylactide, polyglycolide, or a mixed polymer having lactide and glycolide units.

14. Stent according to Claim 12, **characterized in that** the polymer is a biopolymer.

15. Stent according to Claim 14, **characterized in that** the biopolymer is chitin.

16. Stent according to one of the preceding claims, **characterized in that** the polymer contains a medicament.

## Revendications

1. Endoprothèse biorésorbable en un composite métal/polymère, présentant une multitude de segments annulaires (2) et des éléments d'écartement (3) entre les segments annulaires (2), l'endoprothèse (1) présentant une structure en un métal biorésorbable présentant une charge (13) en un polymère biorésorbable, **caractérisée en ce que** la structure métallique présente une multitude d'âmes fendues continues (4), qui sont remplies du polymère (13) pour le renforcement et qui forment un profilé composite avec un noyau polymère (13) entouré par deux joues métalliques (9) de telle sorte que le profilé composite obtient la solidité et la force radiale nécessaires pour remplir la fonction statique, le noyau polymère (13) des âmes (4) représentant 30% à 70% de la largeur de l'âme.

2. Endoprothèse selon la revendication 1, **caractérisée en ce que** âmes (4) présentent des ponts de stabilisation (11) écartés les uns des autres entre les joues (9).

3. Endoprothèse selon la revendication 2, **caractérisée en ce que** les ponts (11) sont disposés au niveau de points d'inflexion et/ou de points de noeud (7, 8) .

4. Endoprothèse selon l'une quelconque des revendications précédentes, présentant une section carrée des âmes (4).

5. Endoprothèse selon l'une quelconque des revendications précédentes, **caractérisée en ce que** l'épaisseur des âmes se situe dans la plage de 60 à 200 µm.

6. Endoprothèse selon l'une quelconque des revendications précédentes, **caractérisée en ce que** les segments annulaires (2) sont formés par des éléments en zigzag, en méandre ou en losange (6).

7. Endoprothèse selon la revendication 6, **caractérisée en ce que** les éléments en losange (6) d'un segment annulaire (2) sont reliés les uns aux autres via les points de noeud (7).

8. Endoprothèse selon la revendication 7, **caractérisée en ce que** les éléments en losange (6) de segments annulaires (2) adjacents sont reliés les uns aux autres au niveau des sommets via des éléments d'écartement (7).

9. Endoprothèse selon la revendication 7 ou 8, **caractérisée en ce que** les points de noeud sont ajourés.

10. Endoprothèse selon l'une quelconque des revendications précédentes, **caractérisée en ce que** le métal est un alliage de magnésium ou du fer pur.

11. Endoprothèse selon la revendication 10, **caractérisée en ce que** le magnésium est allié avec du manganèse et un métal des terres rares.

12. Endoprothèse selon l'une quelconque des revendications précédentes, **caractérisée en ce que** le polymère biorésorbable est un polymère synthétique ou naturel.

13. Endoprothèse selon la revendication 12, **caractérisée en ce que** le polymère est un polylactide, un polyglycolide ou un copolymère pourvu de motifs lactide et glycolide.

14. Endoprothèse selon la revendication 12, **caractérisée en ce que** le polymère est un biopolymère.

15. Endoprothèse selon la revendication 14, **caractérisée en ce que** le biopolymère est la chitine.

16. Endoprothèse selon l'une quelconque des revendications précédentes, **caractérisée en ce que** le polymère contient un médicament.
